# EUROPEAN PATENT SPECIFICATION

(11) **EP 4 398 814 B1**
(45) Date of publication and mention of the grant of the patent: **08.10.2025**
(21) Application number: 22754660.3
(22) Date of filing: 22.07.2022
(51) Int. Cl.: A61B 17/12

(54) **VASO-OCCLUSIVE DEVICE**
GEFÄSSVERSCHLUSSVORRICHTUNG
DISPOSITIF VASO-OCCLUSIF

(30) Priority: 07.09.2021 US 202163241499 P
(43) Date of publication of application: 17.07.2024
(73) Proprietor: Stryker Corporation, Portage, MI 49002-9711 (US); Stryker European Operations Limited, Carrigtwohill, Co. Cork T45 HX08 (IE)
(72) Inventor: CHEN, Hancun, San Ramon, California 94582 (US); LEE, Andrew S., San Jose, California 95124 (US); PARK, Jinhoon, San Jose, California 95131 (US)
(74) Representative: Danubia Patent & Law Office LLC
(86) International application number: PCT/US2022/038058
(87) International publication number: WO 2023/038724

(56) References cited:
- WO-A1-2013/142756
- US-A1- 2015 182 226
- US-A1- 2020 170 647

## Description

### Field of the Invention

The present disclosure relates generally to medical devices and intravascular medical procedures and, more particularly, to devices for occluding vascular defects, such as aneurysms.

### Background

Vaso-occlusive devices or implants are used for a wide variety of reasons, including treatment of intra-vascular aneurysms. An aneurysm is a dilation of a vessel, such as a blood vessel, that may pose a risk to a patient's health due to rupture, clotting, or dissection. For example, rupture of an aneurysm in a patient's brain may cause a stroke, and lead to brain damage and death. Cerebral aneurysms may be detected in a patient, e.g., following seizure or hemorrhage, and may be treated by applying vaso-occlusive devices.

Commonly used vaso-occlusive devices include soft, helically wound coils formed by winding a platinum (or platinum alloy) wire strand about a "primary" mandrel. The coil is then wrapped around a larger, "secondary" mandrel, and heat treated to impart a secondary shape. For example, U.S. Pat. No. 4,994,069, issued to Ritchart et al. describes a vaso-occlusive device that assumes a linear, helical primary shape when stretched for placement through the lumen of a delivery catheter, and a folded, convoluted secondary shape when released from the delivery catheter and deposited in the vasculature. In order to better frame and fill aneurysms, complex three-dimensional secondary shapes can be imparted on vaso-occlusive devices and the stiffness/flexibility of vaso-occlusive devices can be modified.

In order to deliver the vaso-occlusive devices to a desired site in the vasculature, e.g., within an aneurysmal sac, it is well-known to first position a small profile delivery catheter or "micro-catheter" at the site using a guidewire. Typically, the distal end of the micro-catheter is provided, either by the attending physician or by the manufacturer, with a selected pre-shaped bend, e.g., 45°, 26°, "J", "S", or other bending shape, depending on the particular anatomy of the patient, so that it will stay in a desired position for releasing one or more vaso-occlusive device(s) into the aneurysmal sac once the guidewire is withdrawn. A delivery or "pusher" assembly or "wire" is then passed through the micro-catheter until a vaso-occlusive device coupled to a distal end of the delivery assembly is extended out of the distal end opening of the micro-catheter and into the aneurysmal sac. Once in the aneurysmal sac, portions of the vaso-occlusive device may deform or bend to allow more efficient and complete packing. The vaso-occlusive device is then released or "detached" from the distal end of the delivery assembly, and the delivery assembly is withdrawn back through the micro-catheter. Depending on the particular needs of the patient, one or more additional vaso-occlusive devices may be pushed through the micro-catheter and released into the same aneurysmal sac.

Significantly, fluoroscopy is typically used to visualize vaso-occlusive devices during delivery into an aneurysm, while magnetic resonance imaging (MRI) is typically used to visualize the treatment site post-procedure (e.g., a few weeks after initial treatment of the aneurysm) to ensure that the aneurysmal sac is properly occluded. As such, it is important that vaso-occlusive devices be constructed in a manner that enables their radiopacity during treatment of the aneurysm, while minimizing any visualization obscuring artifacts created during the post-procedure MRI (i.e., being MRI-compatible). It is also paramount that such vaso-occlusive devices be "soft" (i.e., be laterally flexible or conformable), and thus atraumatic, to prevent rupturing of the delicate tissues of the aneurysm.

It is also important that such vaso-occlusive devices be chronically retained within the aneurysm. However, aneurysms with larger mouths, commonly known as "wide neck aneurysms," present difficulty in the placement and retention of vaso-occlusive devices within the aneurysm sacs, particularly with small and relatively thin vaso-occlusive coils which lack sufficient mechanical strength to maintain its position within such aneurysm sacs no matter how skillfully they are placed. For this reason, a stent or a balloon must be deployed in the vessel adjacent the neck region of the aneurysm to ensure that the vaso-occlusive coils are retained within the aneurysmal sac, thereby complicating the procedure. To address this particular issue, vaso-occlusive devices at least partially composed of a braided (or woven) structure have been developed. Such braided vaso-occlusive devices provide more neck coverage and a more effective backbone across the necks of aneurysms, and can thus be effectively retained within wide neck aneurysms without the need to deploy supplemental aneurysm-retaining devices, such as balloon or stents.

Vaso-occlusive devices having braided portions may also include coils on the distal and/or proximal end to provide atraumatic ends to the vaso-occlusive device to prevent damage to the fragile tissues of the aneurysm and overall vasculature through which the device is advanced during use. But it has been found that the coils sometimes interact undesirably with the braid by entering its openings between the strands/wires of the braid resulting in interlocking of the braided portion and coil of the vaso-occlusive device. This interlocking condition is also referred to herein as "engagement" or "engaged." When engagement between the coil and the braid occurs, the vaso-occlusive device cannot be maneuvered properly, such as preventing the device from being retracted back into a delivery device (e.g., a delivery catheter), and/or interfering with the proper transition of the vaso-occlusive device from its delivery configuration to its deployed, expanded configuration. The interlocking of the coil with the braid occurs more often when deploying smaller sized vaso-occlusive costs into smaller aneurysm cavities. This is because the limited space within smaller aneurysms constrains the device and forces the coil segment into increased contact with the braid.

Moreover, regardless of whether coiled or braided vaso-occlusive devices are used, conventional vaso-occlusive device delivery systems require that such vaso-occlusive devices be relatively short and limited in expandability, otherwise they are difficult (if not impossible) to push and/or retrieve to/from the microcatheter. Unfortunately, small (short) vaso-occlusive devices are less desirable, since delivery of such small vaso-occlusive devices into an aneurysmal sac may require a longer and more involved procedure. For example, a 7mm diameter neurological aneurysmal sac may typically be filled with five to seven individual spring shaped coils, resulting in a longer and more complicated procedure than if the number of devices was reduced.

Theoretically, the lengths of vaso-occlusive devices may be increased to reduce the number of such vaso-occlusive devices needed to treat an aneurysm. However, increasing the length of a vaso-occlusive device necessarily increases the friction of such vaso-occlusive device with the lumen of the delivery catheter. As such, the columnar strength of such vaso-occlusive device must be increased (e.g., by selecting a material with a high Young's modulus or increasing the diameter of the wire from which the vaso-occlusive device is formed) and/or the diameter of the delivery catheter must be increased to ensure that the vaso-occlusive device can be delivered into the aneurysm. However, as discussed above, it is important that both the diameter of the delivery catheter be as small as possible to allow the aneurysm to be accessed through a very small vasculature, and the vaso-occlusive device be soft enough to prevent trauma to the delicate tissues of the aneurysm.

Materials that enable a relatively long vaso-occlusive device to have the necessary columnar strength to be delivered through a relatively small diameter delivery catheter, while satisfying the other countervailing requirements, including softness, radiopacity, and MRI-compatibility requirements, are very limited. For example, known materials having a relatively high Young's modulus and relatively high radiopacity, such as platinum-tungsten (PtW) alloy from which vaso-occlusive coils are typically manufactured, can be used in an attempt to provide the necessary columnar strength for a relatively long vaso-occlusive device; however, the diameter of the wires from which such vaso-occlusive device is manufactured must be reduced to satisfy the softness requirements while allowing the vaso-occlusive device to fit within a small diameter delivery catheter. As a result, the vaso-occlusive device would have a degraded radiopacity and a decreased columnar strength that would require a shortened vaso-occlusive device and/or larger diameter delivery catheter. International Publication No. WO 2013/142756 A1 discloses an occlusive device 100 for occluding body spaces such as aneurysms. The occlusive device 100 includes a proximal section 102 comprising an expandable, generally tubular braid. The occlusive device 100 also comprises a distal section 104 coupled to a distal end of the proximal section 102. The distal section 104 is an atraumatic tip coil that extends distally form the distal end 110 of the proximal section. This publication does not teach or suggest a vaso-occlusive device in which the relative stiffnesses of the proximal section 104 and distal section 104 are within the range of 0.6 to 0.8 such that the distal section 104 avoids engagement with the braided proximal section 102 during deployment. U.S. Published Patent Application No. US 2015/0182226 A1 discloses a vaso-occlusive device 10 formed by a single filament 16 wound into a primary coil. The vaso-occlusive device 10 has alternating stiffer segments 12 and softer segments 14 along its length. This publication does not teach or suggest any specific range of relative stiffnesses of the stiffer segments 12 and softer segments 14 which avoids engagement of a softer distal segment with a stiffer proximal segment. U.S. Published Patent Application No. US 2020/0170647 discloses a vaso-occlusive structure comprising a mesh portion in which the entirety of the structure comprises a mesh structure having the same stiffness. Again, this publication does not teach or any specific range of relative stiffnesses of the stiffer segments 12 and softer segments 14 which avoids engagement of an atraumatic distal segment with a stiffer proximal segment. Indeed, all of these publications are completely silent as to the problem of engagement of an autramatic distal segment and a braided proximal portion of a vaso-occlusive device.

There, thus, is an ongoing need to provide a vaso-occlusive device that minimizes the problem of interlocking between a coil portion and braid portion, and satisfies the foregoing requirements.

### Summary

The presently claimed invention provides a medical device according to claim 1. Further developments of the herein claimed invention are described in the dependent claims.

In accordance with one aspect of the presently disclosed medical devices, a vaso-occlusive device comprises an elongate vaso-occlusive device (e.g., at least 5cm in length) configured for implantation in an aneurysm sac. The vaso-occlusive device has a delivery configuration when restrained within a delivery catheter and a deployed configuration when released from the delivery catheter into the aneurysmal sac. The vaso-occlusive device includes an elongate braided portion, which comprises the main physical structure of the device. The braided portion is composed of a plurality of elongate strands braided together such that there are gaps or openings between the strands. The braided portion has a proximal end and a distal end. The braided portion has a first bending stiffness, which is a function of the materials, shape, and dimensions of the braid.

The vaso-occlusive device also has a distal coil segment coupled to the distal end of the braided portion. In another aspect, the distal coil segment is typically much shorter than the braided portion, and is used to provide an atraumatic extremity to the device to avoid damaging the delicate tissue of the aneurysm and vasculature during use of the device. The distal coil segment extends distally from the distal end of the braided portion so as to extend the length of the overall device. The distal coil segment has a second bending stiffness.

In another aspect, the ratio of the second bending stiffness (bending stiffness of the distal coil segment) and the first bending stiffness (bending stiffness of the braided portion) is within a range of 0.5 to 1.0, inclusive, or according to the invention, within a range of 0.6 to 0.8, inclusive. This ratio range which has been determined to provide improved performance over previously available vaso-occlusive devices having a braided portion and a distal coil segment. This distal coil segment to braided portion bending stiffness ratio results in an unexpected combination of desirable performance, while also reducing the likelihood of engagement between the distal coil segment and the braided portion. For example, outside of this range, the distal coil segment may be too stiff or too soft resulting in higher chance of engagement and/or undesirable performance characteristics, such as lack of uniformity of loop distribution, more difficult deployment (e.g., requiring more manual manipulation by the clinician), and/or catheter kickback forces causing loss of access to the aneurysm.

Alternatively, the ratio of the second bending stiffness (bending stiffness of the distal coil segment) and the first bending stiffness (bending stiffness of the braided portion) may be within a range of 0.5 to 1.0 or within a range of 0.55 to 0.9, or within a range of 0.65 to 0.75, inclusive, or within any suitable smaller range between 0.6 to 0.8, inclusive, such as 0.68 to 0.72, inclusive.

In still another aspect, the vaso-occlusive device may further comprise a proximal coil segment coupled to the proximal end of the braided portion. The proximal coil segment extends proximally from the proximal end of the braided portion so as to extend the length of the overall device. Similar to the distal coil segment, the proximal coil segment may be much shorter than the braided portion, and provides an atraumatic extremity to the device to avoid damaging the delicate tissue of the aneurysm and vasculature during use of the device. In yet another aspect, the proximal coil segment may have a bending stiffness substantially the same as the second bending stiffness.

In still another aspect, the braided portion of the vaso-occlusive device may have a delivery configuration when restrained within a delivery catheter and deployed configuration when released from a delivery catheter which is different than the delivery configuration. For example, the deployed configuration may be an expanded shape which extends to have a cross-sectional dimension larger than the restrained, delivery configuration. For example, the delivery configuration may be a substantially linear shape or helical coil. The braided portion may be formed of a self-forming/expanding material which is biased to form into the deployed configuration upon release from the delivery catheter. The braided portion may include a shape memory material or component which self-forms upon release, or which forms into the deployed configuration upon exposure to a predetermined condition, such as a change in temperature, an electric current, etc. The deployed configuration may be any suitable shape, such as one or more helical coils, on or more loops, a complex, three-dimensional shape, etc. In another aspect, the deployed configuration is a three-dimensional shape having a cross-sectional dimension of at least three times, or at least 2 times, or at least 1.5 times, the cross-sectional dimension of the delivery configuration.

In accordance with another aspect of the presently disclosed medical devices and intravascular medical procedures, another vaso-occlusive device comprises an elongate main portion having a proximal end and a distal end, and a plurality of openings along a length of the main portion. For example, the main portion may be a braid, a mesh, a tube having a plurality of apertures, or other suitable elongated structure. A tube may be any elongated, hollow object, including without limitation a flat sheet rolled into a tube. The openings are large enough to allow a distal tip of an atraumatic distal segment to enter the openings and engage the main portion. The vaso-occlusive device has a delivery configuration when restrained within a delivery catheter and a deployed configuration when released from the delivery catheter into the aneurysmal sac. The main portion has a first bending stiffness.

The vaso-occlusive device has an atraumatic distal segment coupled to the distal end of the main portion. The distal segment extends distally from the distal end of the main portion. The distal segment has a second bending stiffness. The distal segment has a distal tip wherein the openings are large enough to allow the distal tip to enter the opening and engage the main portion.

The ratio of the second bending stiffness (bending stiffness of the atraumatic distal segment) and the first bending stiffness (bending stiffness of the main portion) is within a range of 0.5 to 1.0, inclusive, or according to the invention, within a range of 0.6 to 0.8, inclusive. Similar to the vaso-occlusive device described above, this ratio range which has been determined to provide improved performance over previously available vaso-occlusive devices having a main portion and an atraumatic distal segment. This distal segment to main portion bending stiffness ratio results in an unexpected combination of desirable performance, while also reducing the likelihood of engagement between the distal segment and the main portion. Beyond this range, the distal segment may be too stiff or too soft resulting in higher chance of engagement and/or undesirable performance characteristics.

Alternatively, the ratio of the second bending stiffness (bending stiffness of the distal segment) and the first bending stiffness (bending stiffness of the main portion) may be within a range of 0.5 to 1.0 or within a range of 0.55 to 0.9, or within a range of 0.65 to 0.75, inclusive, or within any suitable smaller range between 0.6 to 0.8, inclusive, such as 0.68 to 0.72, inclusive.

In another aspect, the distal segment may be a coil, a helical coil, a tube, and a flexible rod, or some combination thereof. The distal segment may have a rounded and/or soft tip to provide an atraumatic extremity for the vaso-occlusive device which avoids damaging the delicate tissue of the aneurysm and vasculature during use of the vaso-occlusive device.

In another aspect, the vaso-occlusive device may also have an atraumatic proximal segment coupled to, and extending proximally from, the proximal end of the main portion. The proximal portion may have any one or more of the same or similar characteristics of the distal segment.

In additional aspects, any of the vaso-occlusive devices disclosed herein may be a part of a vaso-occlusive system comprising a vaso-occlusive assembly and a delivery assembly. For example, a vaso-occlusive assembly may comprise any of the vaso-occlusive devices described herein, and a pusher member detachably coupled to the vaso-occlusive device. The pusher member is configured to allow a clinician to advance the vaso-occlusive device along a delivery catheter through a patient's vasculature to a target site, such as an aneurysm being treated with the vaso-occlusive device, and to push the vaso-occlusive device out of the distal end of the delivery catheter to deploy the vaso-occlusive device.

In still another aspect, the vaso-occlusive assembly may also include a detachment device detachably coupling the pusher member to the vaso-occlusive device. For example, the detachment device may comprise an electrolytic detachment, mechanical connector, heat activated detachment, dissolving detachment, etc. The delivery assembly may include a delivery catheter into which the vaso-occlusive device may be installed in its compact, delivery configuration. The delivery assembly may also include a guidewire for guiding the delivery catheter to a target implantation site within a patient's vasculature, such as an aneurysm. The guidewire is then be removed, and the vaso-occlusive device is advanced through the delivery catheter to the target implantation site.

In still another aspect of the present disclosure, the device is not limited to a vaso-occlusive device, but may be any medical device comprising an elongated main portion and a distal segment attached to a distal end of the main portion, and having the other characteristics of the vaso-occlusive devices disclosed herein. For example, the medical device may be any suitable thrombectomy device, stent retriever, embolic filter, stent delivery system, other implantation device, guidewire, intravascular device, or other medical device. The medical device comprises an elongate main portion having a proximal end and a distal end, and a plurality of openings along a length of the main portion. For example, the main portion may be a braid, a mesh, a tube having a plurality of apertures, or other suitable elongated structure. A tube may be any elongated, hollow object, including without limitation a flat sheet rolled into a tube. The openings are large enough to allow a distal tip of an atraumatic distal segment to enter the openings and engage the main portion. Optionally, the medical device may have a delivery configuration when restrained within a delivery catheter and a deployed configuration when released from the delivery catheter into the aneurysmal sac. The main portion has a first bending stiffness.

The medical device has an atraumatic distal segment coupled to the distal end of the braided portion. The distal segment extends distally from the distal end of the main portion. The distal segment has a second bending stiffness. The distal segment has a distal tip wherein the openings are large enough to allow the distal tip to enter the opening and engage the main portion.

The ratio of the second bending stiffness (bending stiffness of the atraumatic distal segment) and the first bending stiffness (bending stiffness of the main portion) is within a range of 0.5 to 1.0, inclusive, or according to the invention, within a range of 0.6 to 0.8, inclusive. This ratio range provides improved performance over previously available medical devices having a main portion and an atraumatic distal segment. This distal segment to main portion bending stiffness ratio results in an unexpected combination of desirable performance, while also reducing the likelihood of engagement between the distal segment and the main portion. Beyond this range, the distal segment may be too stiff or too soft resulting in higher chance of engagement and/or undesirable performance characteristics.

Alternatively, the ratio of the second bending stiffness (bending stiffness of the distal segment) and the first bending stiffness (bending stiffness of the main portion) may be within a range of 0.5 to 1.0 or within a range of 0.55 to 0.9, or within a range of 0.65 to 0.75, inclusive, or within any suitable smaller range between 0.6 to 0.8, inclusive, such as 0.68 to 0.72, inclusive.

In another aspect, the distal segment may be a coil, a helical coil, a tube, and a flexible rod, or some combination thereof. The distal segment may have a rounded and/or soft tip to provide an atraumatic extremity for the vaso-occlusive device which avoids damaging the delicate tissue of the aneurysm and vasculature during use of the vaso-occlusive device.

Methods of deploying any of the vaso-occlusive devices and other medical devices disclosed herein into an anatomical cavity, such as an aneurysm, are also disclosed although not forming subject of the present application. In one such method, the vaso-occlusive device is inserted into, and advanced through a delivery catheter device in its compact, delivery configuration. The delivery catheter is first inserted into the patient's vasculature and is advanced within the vasculature to position the distal end of the delivery catheter at the target insertion site. In this example, the target insertion site is an aneurysm. It is understood that the target insertion site may be any suitable anatomical site within the vasculature into which the vaso-occlusive device is being deployed. If a guidewire is utilized, the guidewire is first inserted into the patient's vasculature and advanced through the vasculature to the site of the aneurysm. Then, the delivery catheter is advanced along the guidewire to the aneurysm, and then the guidewire is removed.

The vaso-occlusive device is then inserted in its compact, delivery configuration into the delivery catheter and advanced along the delivery catheter until the distal end of the vaso-occlusive device is positioned at the target insertion site. The vaso-occlusive device is then pushed distally out of the delivery catheter using the pusher-member. The distal segment (e.g., distal coil segment) is advanced through the aneurysmal neck and into the aneurysmal sac. As the vaso-occlusive device continues to be advanced out of the delivery catheter via the pusher-member, the braided portion is also advanced into the aneurysmal sac. Also, as the vaso-occlusive device is released from the delivery catheter, it expands into its expanded, deployed configuration within the aneurysmal sac. Once the entire vaso-occlusive device is inserted into the aneurysmal sac, the vaso-occlusive device may be detached from the pusher-member, such as by actuating or activating the detachment device. In some cases, a single vaso-occlusive device may be sufficient to fill and occlude the aneurysm. If multiple vaso-occlusive devices are needed, this process may be repeated to deliver a sufficient number of vaso-occlusive devices to fill and occlude the aneurysm.

Other and further aspects and features of embodiments of the disclosed inventions will become apparent from the ensuing detailed description in view of the accompanying figures.

### Brief Description of the Drawings

The drawings illustrate the design and utility of various aspects of the devices disclosed herein, in which similar elements are referred to by common reference numerals. It should be noted that the figures are not drawn to scale and that elements of similar structures or functions are represented by like reference numerals throughout the figures. It should also be noted that the figures are only intended to facilitate the description of the various aspects of the disclosed technology. They are not intended as an exhaustive description of the technology or as a limitation on the scope of the technology, which is defined only by the appended claims and their equivalents. In addition, an illustrated example of the disclosed technology need not have all the aspects or advantages shown or described herein. An aspect or an advantage described in conjunction with a particular example of the disclosed technology is not necessarily limited to that example and can be practiced in any other examples even if not so illustrated. In order to better appreciate how the above-recited and other advantages and objects of the present technology are obtained, a more particular description of the present technology briefly described above will be rendered by reference to specific examples thereof, which are illustrated in the accompanying drawings. With the understanding that these drawings and corresponding description depict only illustrative examples of the disclosed technology and are not therefore to be considered limiting of its scope, the technology will be described and explained with additional specificity and detail through the use of the accompanying drawings in which:
FIG. 1 is a perspective view of a vaso-occlusive device showing the distal coil segment engaged with the braided portion of the vaso-occlusive device.
FIG. 2A is cross-sectional, side view of a vaso-occlusive system with a vaso-occlusive device in its restrained, delivery configuration within a delivery catheter.
FIG. 2B is a cross-sectional, side view of the vaso-occlusive system of FIG. 1 with the vaso-occlusive device deployed out of the delivery catheter and in its expanded, deployed configuration.
FIG. 3 is a graph showing the relationship between bending stiffness ratio of the distal segment and the main portion of the vaso-occlusive device of FIG. 1 and the performance characteristics and engagement issue for various ranges of bending stiffness ratio.
FIG. 4 is a graph of empirical data for examples of vaso-occlusive devices constructed according to the designs of FIGS. 2A and 2B showing the relationship between bending stiffness ratio of the distal segment and the main portion of the vaso-occlusive device of FIG. 1 and the performance characteristics and engagement issue for various ranges of bending stiffness ratio.
FIG. 5 is a table of empirical data for examples of vaso-occlusive devices constructed according to the designs of FIGS. 2A and 2B and having various bending stiffness ratios.
FIG. 6 is a cross-sectional, side view depicting a guidewire advanced into a portion of a patient's vasculature to the location of an aneurysm.
FIG. 7 is a cross-sectional, side view depicting the guidewire and patient's vasculature of FIG. 6 with a delivery catheter advanced over the guidewire.
FIG. 8 is a cross-sectional, side view depicting the vaso-occlusive system of FIGS. 2A and 2B being advanced within the delivery catheter of FIG. 7 for deploying the vaso-occlusive device into the aneurysm.
FIG. 9 is a cross-sectional, side view depicting the vaso-occlusive system of FIG. 7 with the vaso-occlusive device deployed into the aneurysm.
FIG. 10 is a flow chart of using the vaso-occlusive system of FIGS. 2A and 2B to deploy the vaso-occlusive device into the aneurysm.

### Detailed Description

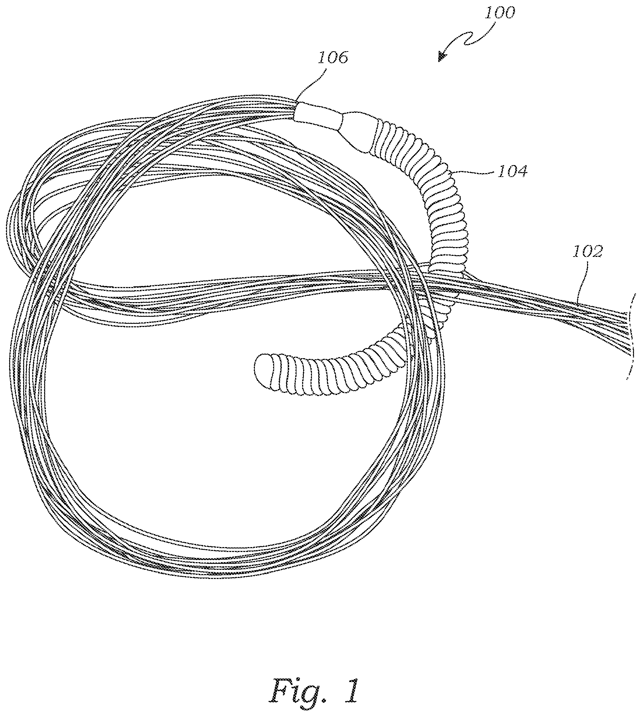

FIG. 1 illustrates the engagement problem of a vaso-occlusive device 100 comprising a braided portion 102 and a distal coil segment 104. The vaso-occlusive device 100 comprises a main, braided portion 102 and a distal coil segment 104 attached to the distal end 106 of the braided portion 102. It has been discovered that during insertion of such vaso-occlusive devices 100 comprising a braided portion 102, or other portion having openings sufficiently large to receive the distal coil segment 104, the distal coil segment 104 can become engaged in the braided portion 102 as the vaso-occlusive device 100 transitions from its delivery configuration within a delivery catheter to an expanded, deployed configuration as it is advanced out of the delivery catheter and into an anatomical cavity, such as an aneurysm. This engagement is shown in FIG. 1 which depicts the vaso-occlusive device 100 in a deployed configuration having a three-dimensional shape. The distal coil segment 104 has become inserted through an opening in the braided strands of the braided portion 102, as shown in FIG. 1, and such that the distal coil segment 104 is firmly engaged with the braided portion. This is a very undesirable outcome because the engagement prevents the vaso-occlusive device 100 from being maneuvered properly, such as preventing the device 100 from being retracted back into a delivery device (e.g., a delivery catheter), and/or interfering with the proper transition of the vaso-occlusive device 100 from its delivery configuration to its deployed, expanded configuration.

Although the engagement problem and specific examples are described herein with respect to a vaso-occlusive device for occluding an anatomical space (such as an aneurysm), the present disclosure is not limited to such device, but includes any medical device comprising an elongated main portion and a distal segment attached to a distal end of the main portion. For example, the medical device may be any suitable thrombectomy device, stent retriever, embolic filter, stent delivery system, other implantation device, guidewire, intravascular device, or other medical device.

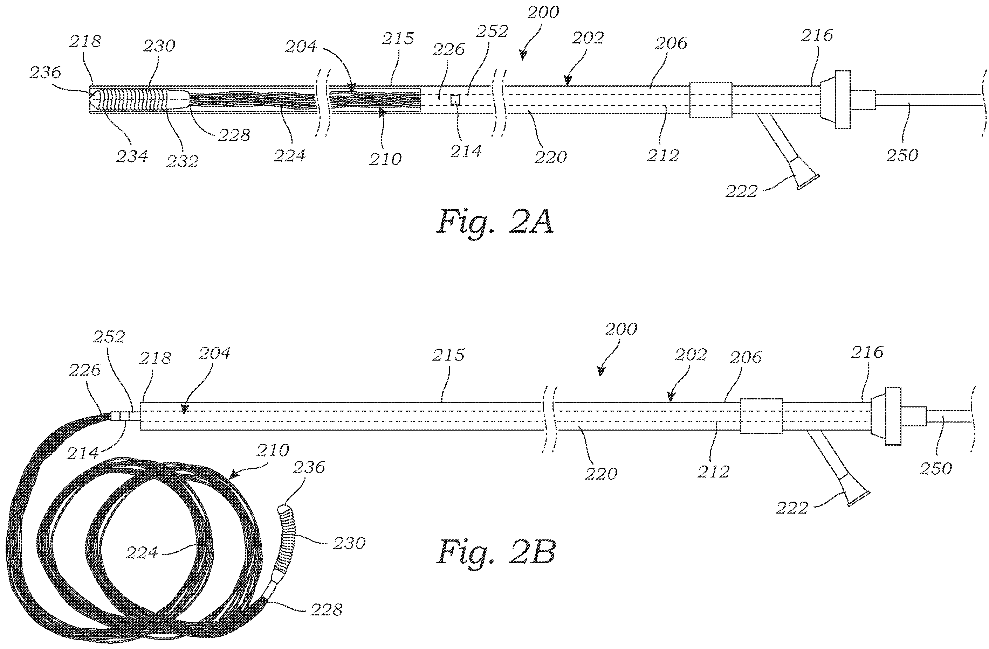

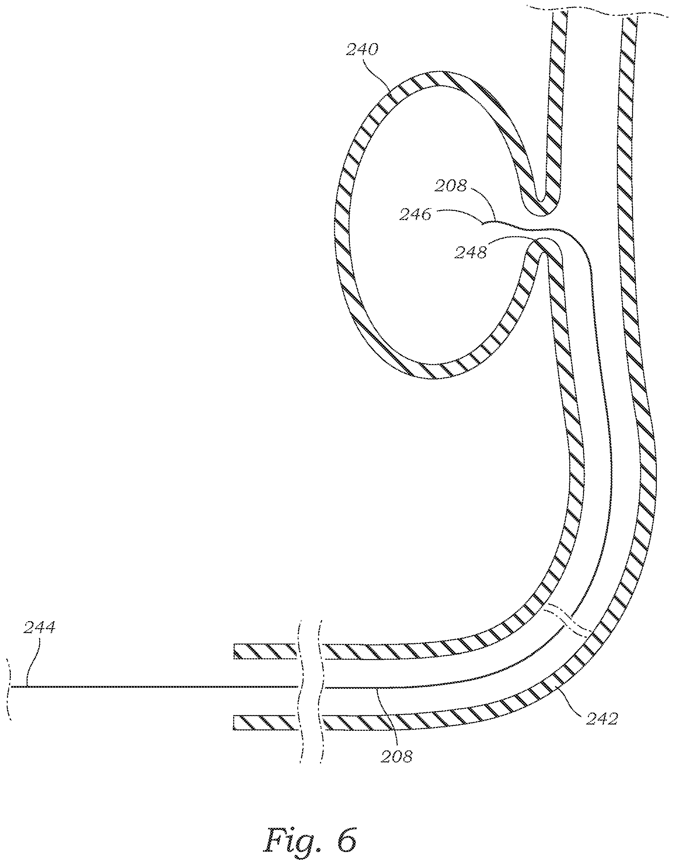

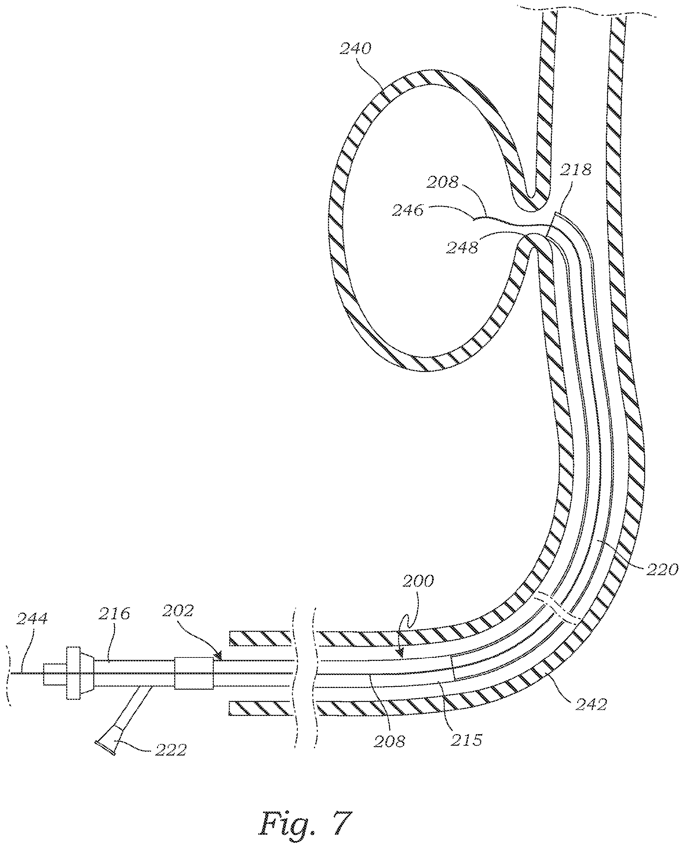

Referring to FIGS. 2A and 2B, a vaso-occlusive system 200 having a vaso-occlusive device 210 which alleviates the engagement problem is illustrated. The vaso-occlusive device 210 also exhibits good performance characteristics during insertion and use. The vaso-occlusive system 200 comprises a delivery assembly 202 and a vaso-occlusive assembly 204. As shown in FIGS. 6 and 7, the delivery assembly 202 may include a delivery catheter 206 and an optional guidewire 208. The vaso-occlusive assembly 204 comprises a vaso-occlusive device 210 and a pusher member 212 detachably coupled to the vaso-occlusive device 210 via a detachment device or junction 214. FIG. 2A shows the vaso-occlusive assembly 204 after it has been slidably disposed within the delivery catheter 206 such that the vaso-occlusive device is in its compact, delivery configuration.

The delivery catheter 206 is typically an elongated, flexible tube, and can be, for example, a microcatheter or the like. The delivery catheter 206 comprises an elongate sheath body 215 having a proximal portion 216, a distal portion 218 and a lumen 220 extending from the proximal portion 216 to the distal portion 218. The proximal portion 216 of the delivery catheter 206 typically remains outside of the patient and accessible to the clinician when the vaso-occlusive system 200 is used, while the distal portion 218 is sized and dimensioned to reach remote locations of a patient's vasculature and is configured to deliver the vaso-occlusive device 210 to an aneurysm. The delivery catheter 206 may also have one or more ports 222 in fluid communication with the lumen 220 for introducing into, or removing fluids from, the sheath body 215. The sheath body 215 may be composed of suitable polymeric materials, metals and/or alloys, such as polyethylene, stainless steel or other suitable biocompatible materials or combinations thereof. In some instances, the proximal portion 216 may include a reinforcement layer, such as a braided layer or coiled layer to enhance the pushability of the sheath body 215. The sheath body 215 may include a transition region between the proximal portion 216 and the distal portion 218.

The vaso-occlusive device 210 comprises an elongated, main portion 224 having a proximal end 226 and a distal end 228. The main portion 224 may be a braided portion comprising a plurality of strands braided together to form a resilient, tube-shaped member. Alternatively, the main portion 224 may comprise a mesh, or a tube (a tube may be any elongated, hollow object, including without limitation a flat sheet rolled into a tube) having a plurality of apertures, or other elongate structure having a plurality of openings along a length of the main portion 224, such as along substantially the entire length of the main portion 224, or at least 50% of the length of the main portion 224, or at least 75% of the length of the main portion. The main portion 224 has a first bending stiffness which is a measure of the main portion's 224 resistance to bending deformation, as is typically expressed in a bending moment per unit width, such as in units of "mN/mm". In the case of a braided portion 224, the first bending stiffness is determined by the braid configuration and its secondary diameter (i.e., the braid portion 224 OD or device 210 OD).

The vaso-occlusive device 210 also has a flexible, atraumatic distal segment 230 coupled to the distal end 228 of the main portion 224. The distal segment 230 has a proximal end 232 and a distal end 234. The distal segment 230 in the figures comprises a helical coil such that the distal segment 230 is a distal coil segment 230. The proximal end 232 of the distal coil segment 230 may be attached to the distal end 228 of the main portion 224 by any suitable means, such as welding, mechanical fastener, adhesive, etc. The distal segment 230 may also have an atraumatic tip 236 attached to the distal end 234 of the helical coil 230. The atraumatic distal segment 230 has a second bending stiffness which is lower than the first bending stiffness of the main portion 224. The distal segment 230 is more flexible, i.e., has a lower bending stiffness, than the first bending stiffness of the main portion 224 so as to provide an atraumatic distal tip for the vaso-occlusive device which does not damage, rupture, or otherwise cause trauma to, the delicate tissues of the aneurysm and/or vasculature as the vaso-occlusive device 210 is advanced out of the delivery catheter 206 into the patient's vasculature and aneurysm. Alternatively, the distal segment 230 may be other suitable flexible structures which provide the desirable atraumatic characteristics, such as a polymer rod or tube, etc.

The atraumatic distal segment 230 has a second bending stiffness, different than the first bending stiffness of the main portion 224. In the case of a distal coil segment 230, the second bending stiffness is a function of the coil wire diameter, pitch, primary coil diameter and secondary coil diameter (secondary coil shape diameter). As described herein, certain ranges of the ratio of the second bending stiffness of the distal segment 230 and the first bending stiffness of the main portion 224 (referred to herein as the "bending stiffness ratio") can mitigate the engagement problem, while also providing desirable performance characteristics of the vaso-occlusive device 210. For a given first bending stiffness of the main portion 224, the second bending stiffness of the distal coil segment 230 can be manipulated by controlling the distal segment 230 configuration together with the OD of the distal segment 230 to target a desired range of the ratio of the second bending stiffness and the first bending stiffness.

The vaso-occlusive device 210 is sized for implantation in an aneurysmal sac 240 (see FIGS. 6-9), which can take any geometry or shape in its cross-section. For example, in the illustrated embodiment of FIGS. 1 and 2, the vaso-occlusive device 210 takes the form of a resilient, braided, mesh or porous main portion 224 having tubular shape which may be flattened in portions due to internal and/or external forces, and a helical coil, distal segment 230.

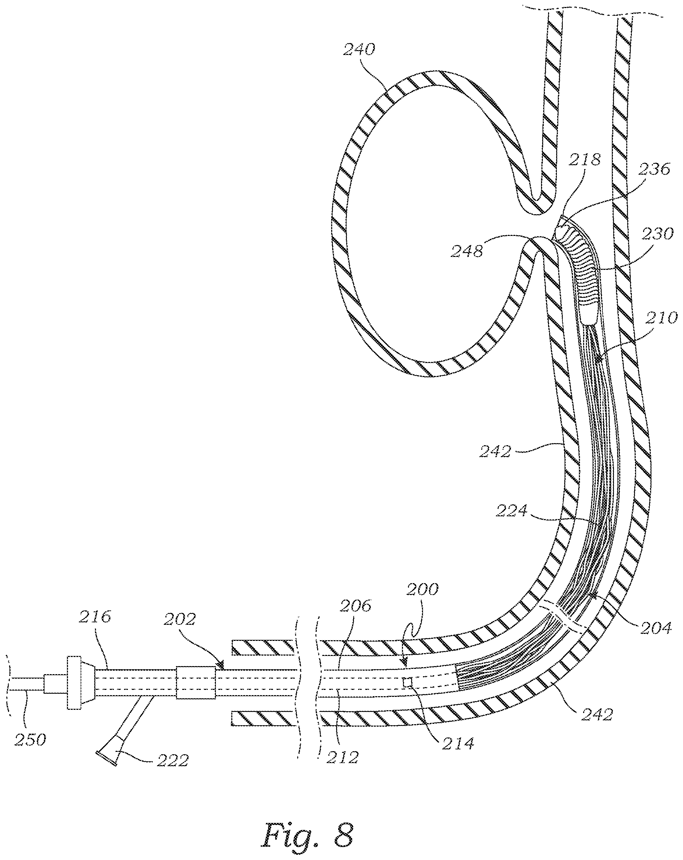

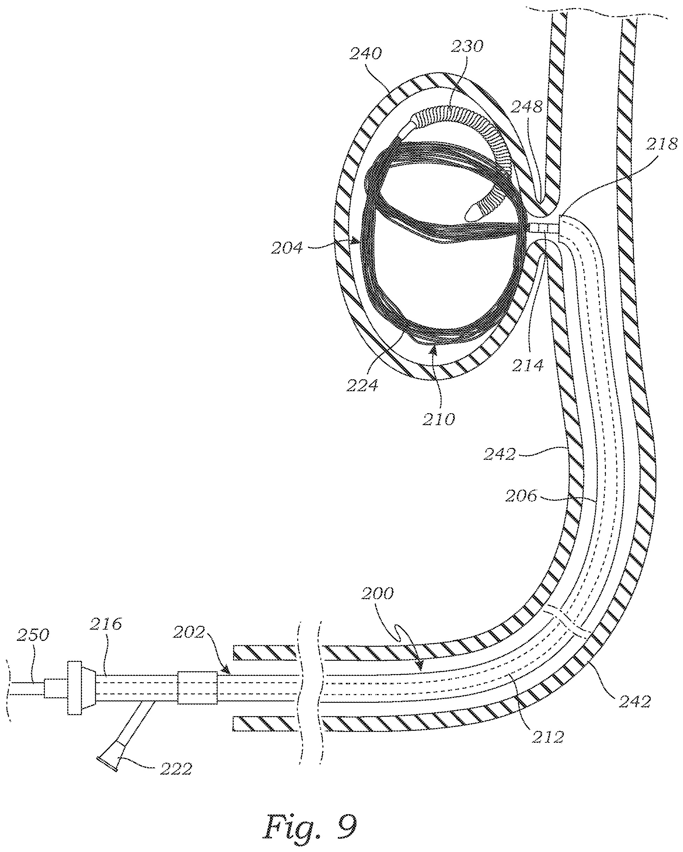

Optionally, the vaso-occlusive device 210 may have a compact delivery configuration when radially restrained within the delivery catheter 206 and may be configured to form into a deployed configuration having a secondary shape different than the delivery configuration when released from the delivery catheter 206, such as release into the aneurysmal sac 240 or other anatomical cavity. FIG. 2A shows the vaso-occlusive device 210 in its compact delivery configuration in which it is retrained within the delivery catheter 206 and substantially conforms to the longitudinal path (i.e., shape) of the delivery catheter 206. For instance, in FIG. 2A, the vaso-occlusive device 210 has a substantially linear axial shape. As shown in FIG. 8, when inserted into the delivery catheter 206, the vaso-occlusive device 210 tracks the longitudinal path of the delivery catheter 206 within the vascular vessel 242. As shown in FIGS. 2B and 9, upon release from the delivery catheter 206, the vaso-occlusive device 210 forms into its deployed configuration, which is a three-dimensional shape comprising a plurality of loops and/or curves which may be non-overlapping, overlapping, or a combination thereof. The cross-sectional dimension of the vaso-occlusive device 210, in its deployed configuration, may, e.g., be greater than 1.5 times, and preferably greater than 2 times, and most preferably, greater than 3 times, the cross-sectional dimension of the vaso-occlusive device 210 in its compact delivery configuration.

The deployed configuration of the vaso-occlusive device 210 may be formed or programmed into the device by any suitable method. For example, the main portion 224 (e.g., a braided main portion 224) may be formed of a shape memory material. The main portion 224, such as a braid 224, may be formed of any suitable material, including, without limitation, a platinum alloy, a platinum-tungsten alloy, a gold alloy, or nitinol, or combinations of any of the foregoing. The main portion 224 is wound around a mandrel in the shape of the loops and/or curves of the deployed configuration, and then heated treated to form or program the deployed configuration into the main portion 224. Alternatively, the main portion 224 may be formed of a shape memory material and programmed to take on the deployed configuration upon exposure of the main portion 224 to a predetermined condition, such as a change in temperature, an electric current, or other shape forming condition.

As described herein, the main portion 224 may be formed of a braid having a desired length (e.g., greater than 5 cm, between 5 cm and 45 cm, between 5 cm and 30 cm, etc. The braid may be formed from a plurality of wires or strands using braiding machines, and may be braided around a mandrel (e.g., a mandrel having a round, oval, flat, other shape depending on the desired final cross-sectional shape of the main portion 224). After braiding, the main portion 224 can be heat set into its delivery configuration to form a linear "primary shape" of the mesh portion 224. The heat set completed braid can then be wrapped around a second mandrel (e.g., a three-dimensional mandrel) and heat set for a second time to impart the three-dimensional deployed configuration.

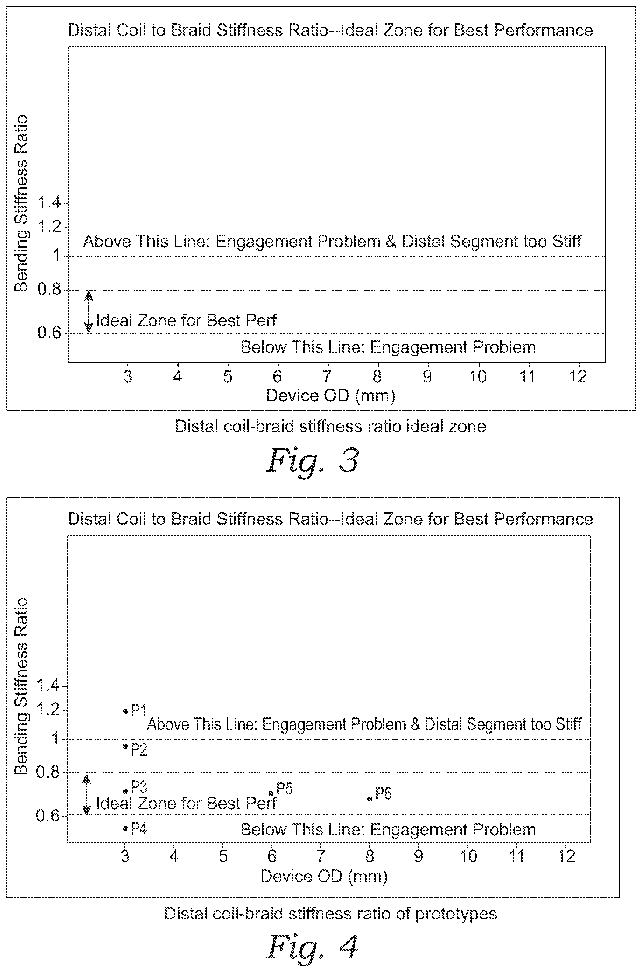

Turning to FIG. 3, the graph illustrates the optimized combination of desirable performance characteristics and minimized engagement for the vaso-occlusive device 210 for various ranges of bending stiffness ratio. The graph of Fig. 3 plots the bending stiffness ratio against the main portion 224 OD (device OD or braid portion secondary shape OD). The performance characteristics of the vaso-occlusive device 210 include observable functional attributes such as uniformity of loop distribution within an aneurysm, ease of deployment, effective seeding of the distal segment 230 within the aneurysm (i.e., during deployment, the distal segment 230 remains within the aneurysm and does not steer back out of the aneurysm), and catheter kickback forces during deployment (i.e., the amount of kickback force exerted on the delivery catheter 206 causing displacement of the distal end 218 of the delivery catheter 206).

The graph of FIG. 3 shows that above a bending stiffness ratio of 1.0, the vaso-occlusive device 210 has a high degree of engagement and the distal segment is too stiff causing undesirable performance, including lack of uniformity of loop distribution within the aneurysm, difficulty in deployment requiring increased user input (e.g., manipulation) to deploy the vaso-occlusive device 210, inconsistent seeding of the distal segment 230, and/or catheter kickback causing the distal end 218 of the delivery catheter 206 to move away from the target insertion site.

Still referring to FIG. 3, a bending stiffness ratio in the range of 0.8 to 1.0, the vaso-occlusive device 210 still exhibits undesirable performance characteristics, although it tends to eliminate the engagement problem. More particularly, the vaso-occlusive device 210 having a bending stiffness ratio in the range of 0.8 to 1.0 results in a lack of uniformity of loop distribution, difficulty in deployment, requiring increased user input (e.g., manipulation) to deploy the vaso-occlusive device 210, and/or catheter kickback causing the distal end 218 of the delivery catheter 206 to move away from the target insertion site.

FIG. 3 shows that a bending stiffness ratio for the vaso-occlusive device 210 in the range of 0.6 to 0.8, inclusive, provides the best combination of desirable performance characteristics and avoidance of engagement. The bending stiffness range of 0.6 to 0.8 provides a vaso-occlusive device 210 which results in uniform loop distribution, ease of deployment requiring minimal user input during deployment, consistent seeding of the distal segment 230 without egress from the aneurysm, and minimal catheter kickback allowing the distal end 218 of the delivery catheter 206 to remain in proper position at the target insertion site, such as within the neck of the aneurysm.

As shown in FIG. 3, a bending stiffness ratio for the vaso-occlusive device 210 below 0.6 results in excessive engagement during deployment, although the vaso-occlusive device 210 exhibits desirable other performance characteristics, such as good uniformity of loop distribution, ease of deployment, consistent seeding, and minimal catheter kickback. However, due to the excessive engagement, the vaso-occlusive device 210 having a bending stiffness ratio below 0.6 is not desirable.

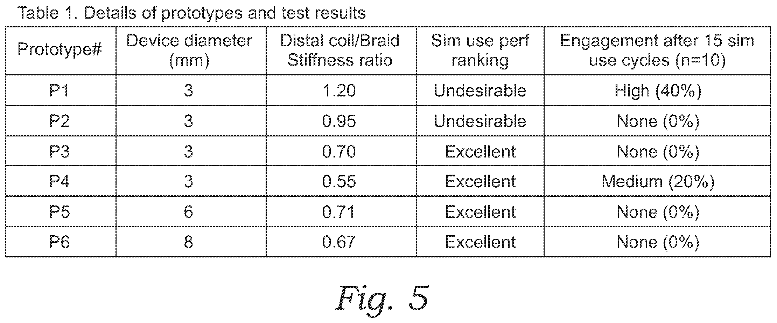

FIGS. 4 and 5 provide empirical, experimental data for several examples of prototypes of vaso-occlusive devices 210 constructed according to the devices 210 illustrated in FIGS. 1 and 2 and described herein. The experimental data was used to analyze the effect of the bending stiffness ratio on the engagement issue and performance characteristics, and to determine the optimal bending stiffness range. The different prototypes are labeled as P1-P6 in the graph of FIG. 4 and Table 1 of FIG. 5. Prototypes P1 have a bending stiffness ratio of 1.20. The testing was performed for 15 simulated use cycles and 10 samples of each prototype design. As shown in FIGS. 4 and 5, prototypes P1 demonstrated undesirable performance characteristics and high incidence of engagement (40%), as described herein for a bending stiffness ratio above 1.0.

Prototypes P2 have a bending stiffness ratio of 0.95, and demonstrated undesirable performance characteristics, with no engagement issues, as described herein for a bending stiffness ratio in the range of 0.8 to 1.0. Prototypes P3 have a bending ratio of 0.70, prototypes P5 have a bending ratio of 0.71, and prototypes P6 have a bending ratio of 0.67. Prototypes P3, P5 and P6 demonstrated both excellent performance characteristics, and no engagement issues, as described herein for a bending stiffness ratio in the range of 0.6 to 0.8, inclusive. Prototypes P3 (vaso-occlusive device 210 diameter OD of 3 mm), P5 (vaso-occlusive device 210 diameter OD of 6 mm) and P6 (vaso-occlusive device 210 diameter OD of 8 mm) also demonstrate that differences in the vaso-occlusive device 210 diameter do not have a significant effect on the performance characteristics and/or engagement issue.

Prototypes P4 have a bending stiffness ratio of 0.55, and while demonstrating excellent performance characteristics, prototypes P4 exhibited a medium degree of engagement problems with the distal segment 230 engaging the main portion 224 in 20% of the simulate use cycles, as described herein for a bending stiffness ratio below 0.60.

Alternatively, the bending stiffness ratio may be within a range of 0.55 to 0.9, or within a range of 0.65 to 0.75, inclusive, or within any suitable smaller range between 0.6 to 0.8, inclusive, such as 0.68 to 0.72, inclusive.

Referring back to FIGS. 2A and 2B, the vaso-occlusive assembly 204 also comprises a pusher member 212. The pusher member 212 is disposed within the lumen 220 of the delivery catheter 206. The pusher member 212 has a proximal portion 250, which typically extends proximal of the proximal portion 216 of the delivery catheter 206, and a distal portion 252 which is detachably coupled to the proximal portion 226 of the vaso-occlusive device 210 via the detachment device 214. The pusher member 212 may be a coil, wire, tendon, conventional guidewire, torqueable cable tube, hypotube, or the like, having a sufficient columnar strength to permit pushing of the vaso-occlusive device 210 out through distal end 218 of the delivery catheter 206 and into the aneurysmal sac 240 (see FIGS. 8 and 9).

The detachment device 214 provides a detachable connection between the pusher member 212 and the vaso-occlusive device 210. The detachment device 214 may comprise an electrolytically detachment, mechanical connector, heat activated detachment, dissolving detachment, or other mechanical, thermal and hydraulic mechanism. For instance, the detachment device 214 may be an electrolytically degradable segment for electrolytically decoupling the vaso-occlusive device 210 from the pusher member 212.

As shown in FIGS. 6 and 7, the optional guidewire 208 of the delivery assembly 202 has a proximal end 244 and a distal end 246. As shown in FIG. 7, after the guidewire 208 is positioned within the patient's vasculature 242 with the distal end 246 located at the target insertion site, the delivery catheter 206 is advanced over the guidewire 208 with the guidewire 208 disposed within the lumen 220 of the delivery catheter 206. In a "rapid-exchange" configuration of the delivery catheter 206 and guidewire 208, the guidewire 208 extends through only a distal portion of the delivery catheter 206, such as a rapid-exchange lumen. The guidewire 208 is typically used by first advancing the guidewire 208 through the patient's vasculature to the target insertion site (e.g., the neck of an aneurysm to be filled by the vaso-occlusive device 210), and then advancing the delivery catheter 206 over the guidewire 208 to the target insertion site.

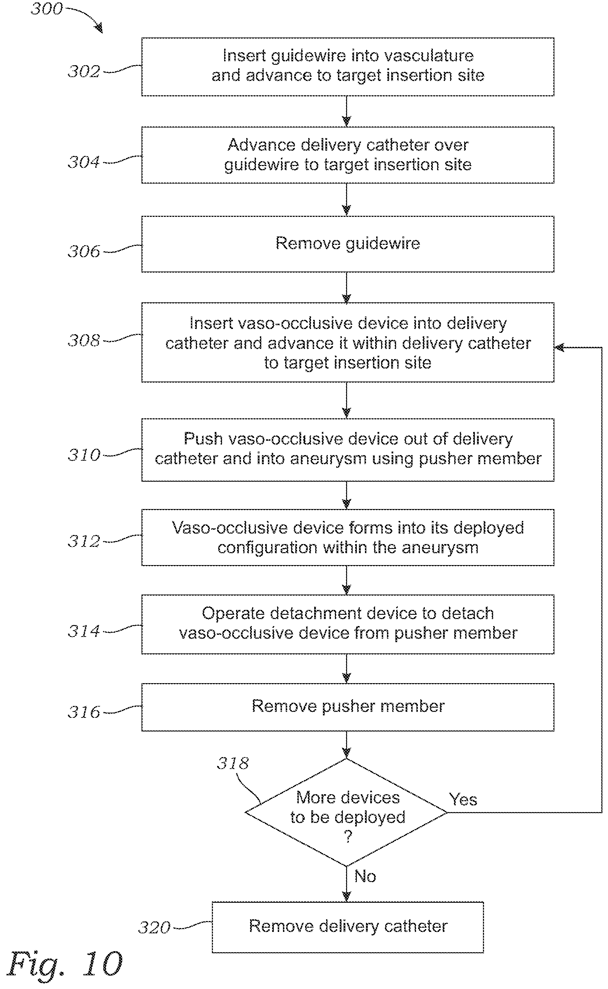

Turning to FIGS. 6-10, an exemplary method 300 of using the vaso-occlusive system 200 to deploy the vaso-occlusive device 210 into an anatomical cavity will now be described as an illustration. This will be described with respect to deploying the vaso-occlusive device 210 into an aneurysmal sac 240, as an example. However, the method 300, as an illustration is not limited to deploying the vaso-occlusive device 210 into an aneurysmal sac 240, but may be used to deploy the vaso-occlusive device 210, or other medical device as disclosed herein, into any suitable anatomical cavity which is accessible via a patient's vasculature. Referring to the flow chart of FIG. 10, at step 302, the guidewire 208 is inserted into the patient's vasculature 242 and is advanced to the target insertion site, namely the aneurysmal sac 240. As described herein, the use of the guidewire 208 is optional, and is not required in the method 300 of using the vaso-occlusive system 200 to deploy the vaso-occlusive device 210.

At step 304, the delivery catheter 206 of the delivery assembly 202 is advanced over the guidewire 208 until it is positioned with the open distal end 218 adjacent or within the aneurysmal neck 248 of the aneurysm, as shown in FIG. 7. At step 306, the guidewire 208 is pulled out of the delivery catheter 206 leaving the delivery catheter 206 in position. At step 308, the vaso-occlusive assembly 204 is inserted into the delivery catheter 206 of the delivery assembly 202 and advanced within the delivery catheter 206 to position the distal end 234 of the vaso-occlusive device 210 adjacent the distal portion 218 of the delivery catheter 206. At this position, the proximal portion 250 of the pusher member 212 remains proximal and outside of the proximal portion 216 of the delivery catheter 206. In one aspect of the method 300, provided as an illustration, prior to inserting the vaso-occlusive device 210 into the delivery catheter 206, the vaso-occlusive device 210 is pre-installed in a sheath such that the vaso-occlusive device 210 is in its delivery configuration. The vaso-occlusive device 210 is then inserted into the delivery catheter 206 by placing a distal end of the sheath in abutment with the proximal end 216 of the delivery catheter 206 and extruding the vaso-occlusive device 210 from the sheath into the delivery catheter 206 such that the vaso-occlusive device 206 remains in its delivery configuration within the delivery catheter 206.

At step 310, the vaso-occlusive device 210 is pushed through the lumen 220 of the delivery catheter 206 and distally out of the delivery catheter 206 by pushing on the proximal portion 250 of the pusher member 212. As the vaso-occlusive device 210 is pushed out of the open distal end 218 of the delivery catheter 206, the distal segment 230 is advanced through the aneurysmal neck 248 and into the aneurysmal sac 240 thereby seeding the vaso-occlusive device 210 within the aneurysmal sac 240. As the vaso-occlusive device 210 continues to be advanced out of the delivery catheter 206 via the pusher member 212, the main portion 224 is also advanced into the aneurysmal sac 240. Also, as the vaso-occlusive device 210 is released from the delivery catheter 206, at step 312, the vaso-occlusive device 210 forms into its deployed configuration within the aneurysmal sac 240. Once the entire vaso-occlusive device 210 is inserted into the aneurysmal sac 240, at step 314, the detachment device 214 is actuated, activated or otherwise operated to detach the vaso-occlusive device 210 from the pusher-member 212. At step 316, the pusher member 212 is removed from the patient's vasculature 242 by withdrawing it out through the delivery catheter 206. If the single vaso-occlusive device 210 is sufficient to fill and occlude the aneurysm sac 240, then the method 300 proceeds to step 320 in which the delivery catheter 206 is removed from the patient's vasculature 242. 308-316, a decision is made whether more additional vaso-occlusive devices 210 are to be deployed. If multiple vaso-occlusive devices 210 are being implanted, then the process of steps 308-318 are repeated to deliver a sufficient number of vaso-occlusive devices 210 to fill and occlude the aneurysmal sac 240. After the sufficient number of vaso-occlusive devices 210 are implanted in the aneurysmal sac 240, the delivery catheter 206 is removed at step 320.

## Claims

1. A medical device (210) comprising:
an elongate main portion (224) having a proximal end (226) and a distal end (228), the main portion (224) having a first bending stiffness, the main portion (224) having plurality of openings along a length of the main portion (224);
an atraumatic distal segment (230) coupled to, and extending distally from, the distal end (228) of the main portion (224), the distal segment (230) having a second bending stiffness, the distal segment (230) having a distal tip (236); and
wherein the plurality of openings of the main portion are large enough to allow the distal tip (236) to enter the openings and engage the main portion (224);
**characterized in that**,
the ratio of the second bending stiffness to the first bending stiffness is within the range of 0.6 to 0.8, inclusive, such that the distal segment (230) avoids engagement with the openings of the main portion (224) during deployment.

2. The medical device of claim 1, wherein:
the plurality of openings extends along at least 50% of the length of the main portion (224).

3. The medical device of claim 1, wherein the ratio of the second bending stiffness to the first bending stiffness is within the range of 0.65 to 0.75, inclusive.

4. The medical device of any of claims 1-3, wherein the elongate main portion (224) comprises one a braid, a mesh, and a tube having a plurality of apertures.

5. The medical device of claim 1, wherein the distal segment (230) is one of a coil, a helical coil, a tube, a braid and a flexible rod.

6. The medical device of claim 1, further comprising:
an atraumatic proximal portion coupled to, and extending proximally from, the proximal end of the main portion.

7. The medical device of claim 1, wherein the medical device is a vaso-occlusive device.

8. The vaso-occlusive device (210) of claim 1,
wherein the main portion (224) comprises an elongate braided portion (224) comprising a plurality of elongate strands braided together, and the distal segment comprises a distal coil segment; and
and the plurality of openings comprise openings in the braided portion (224).

9. The vaso-occlusive device of claim 8, wherein:
the plurality of openings extends along at least 50% of the length of the braided portion.

10. The vaso-occlusive device of claim 8, further comprising:
a proximal coil segment coupled to, and extending proximally from, the proximal end of the braided portion.

11. The vaso-occlusive device of claim 8, wherein the braided portion (224) has a delivery configuration when restrained within a delivery catheter (206) and a deployed configuration when released from a delivery catheter (204).

12. The vaso-occlusive device of claim 11, wherein the delivery configuration is a substantially linear shape, and the deployed configuration is a three-dimensional shape having a cross-sectional dimension of at least three times the cross-sectional dimension of the delivery configuration.

13. The vaso-occlusive device of claim 8, wherein the braided portion (224) is formed of a shape memory material.

14. The vaso-occlusive device of claim 8, wherein the braided portion (224) is formed of one of platinum, a platinum alloy, and a platinum-tungsten alloy.

15. The vaso-occlusive device of claim 8, wherein the braided portion (224) is formed of one of gold, and a gold alloy.

16. The vaso-occlusive device of claim 8, wherein the braided portion (224) is formed of one of a platinum-gold alloy, and nitinol.

17. A vaso-occlusive assembly (200), comprising:
the vaso-occlusive device (210) of claim 8; and
a pusher member (212) detachably coupled to the vaso-occlusive device (210).

18. The vaso-occlusive assembly of claim 17, further comprising:
a detachment device (214) detachably coupling the pusher member (212) to the vaso-occlusive device (210).

19. The vaso-occlusive assembly of claim 18, wherein the detachment device (214) comprises one of electrolytic, mechanical connector, heating and dissolving.

## Patentansprüche

1. Medizinische Vorrichtung (210), umfassend:
einen länglichen Hauptabschnitt (224) mit einem proximalen Ende (226) und einem distalen Ende (228), wobei der Hauptabschnitt (224) eine erste Biegesteifigkeit aufweist, wobei der Hauptabschnitt (224) eine Vielzahl von Öffnungen entlang einer Länge des Hauptabschnitts (224) aufweist;
ein atraumatisches distales Segment (230), das mit dem distalen Ende (228) des Hauptabschnitts (224) gekoppelt ist und sich distal davon erstreckt, wobei das distale Segment (230) eine zweite Biegesteifigkeit aufweist, wobei das distale Segment (230) eine distale Spitze (236) aufweist; und
wobei die Vielzahl von Öffnungen des Hauptabschnitts groß genug ist, um zu ermöglichen, dass die distale Spitze (236) in die Öffnungen eintritt und in den Hauptabschnitt (224) eingreift;
**dadurch gekennzeichnet, dass**
das Verhältnis der zweiten Biegesteifigkeit zur ersten Biegesteifigkeit innerhalb des Bereichs von einschließlich 0,6 bis einschließlich 0,8 liegt, sodass das distale Segment (230) den Eingriff mit den Öffnungen des Hauptabschnitts (224) während des Einsatzes vermeidet.

2. Medizinische Vorrichtung nach Anspruch 1, wobei:
sich die Vielzahl von Öffnungen entlang mindestens 50 % der Länge des Hauptabschnitts (224) erstreckt.

3. Medizinische Vorrichtung nach Anspruch 1, wobei das Verhältnis der zweiten Biegesteifigkeit zur ersten Biegesteifigkeit innerhalb des Bereichs von einschließlich 0,65 bis einschließlich 0,75 liegt.

4. Medizinische Vorrichtung nach einem der Ansprüche 1-3, wobei der längliche Hauptabschnitt (224) eines von einem Geflecht, einem Netz und einem Rohr mit einer Vielzahl von Öffnungen umfasst.

5. Medizinische Vorrichtung nach Anspruch 1, wobei das distale Segment (230) eines von einer Spule, einer spiralförmigen Spule, einem Rohr, einem Geflecht und einem flexiblen Stab ist.

6. Medizinische Vorrichtung nach Anspruch 1, ferner umfassend:
einen atraumatischen proximalen Abschnitt, der mit dem proximalen Ende des Hauptabschnitts gekoppelt ist und sich proximal davon erstreckt.

7. Medizinische Vorrichtung nach Anspruch 1, wobei die medizinische Vorrichtung eine vasookklusive Vorrichtung ist.

8. Vasookklusive Vorrichtung (210) nach Anspruch 1,
wobei der Hauptabschnitt (224) einen länglichen geflochtenen Abschnitt (224) umfasst, der eine Vielzahl von länglichen Strängen umfasst, die zusammengeflochten sind, und das distale Segment ein distales Spulensegment umfasst; und
und die Vielzahl von Öffnungen Öffnungen in dem geflochtenen Abschnitt (224) umfasst.

9. Vasookklusive Vorrichtung nach Anspruch 8, wobei:
sich die Vielzahl von Öffnungen entlang mindestens 50 % der Länge des geflochtenen Abschnitts erstreckt.

10. Vasookklusive Vorrichtung nach Anspruch 8, ferner umfassend:
ein proximales Spulensegment, das mit dem proximalen Ende des geflochtenen Abschnitts gekoppelt ist und sich proximal davon erstreckt.

11. Vasookklusive Vorrichtung nach Anspruch 8, wobei der geflochtene Abschnitt (224) eine Zuführungskonfiguration, wenn er innerhalb eines Zuführungskatheters (206) zurückgehalten wird, und eine entfaltete Konfiguration, wenn er von einem Zuführungskatheter (204) freigegeben wird, aufweist.

12. Vasookklusive Vorrichtung nach Anspruch 11, wobei die Zuführungskonfiguration eine im Wesentlichen lineare Form ist und die entfaltete Konfiguration eine dreidimensionale Form mit einer Querschnittsabmessung von mindestens dem Dreifachen der Querschnittsabmessung der Zuführungskonfiguration ist.

13. Vasookklusive Vorrichtung nach Anspruch 8, wobei der geflochtene Abschnitt (224) aus einem Formgedächtnismaterial gebildet ist.

14. Vasookklusive Vorrichtung nach Anspruch 8, wobei der geflochtene Abschnitt (224) aus einem von Platin, einer Platinlegierung und einer Platin-Wolfram-Legierung gebildet ist.

15. Vasookklusive Vorrichtung nach Anspruch 8, wobei der geflochtene Abschnitt (224) aus einem von Gold und einer Goldlegierung gebildet ist.

16. Vasookklusive Vorrichtung nach Anspruch 8, wobei der geflochtene Abschnitt (224) aus einem von einer Platin-Gold-Legierung und Nitinol gebildet ist.

17. Vasookklusive Anordnung (200), umfassend:
die vasookklusive Vorrichtung (210) nach Anspruch 8; und
ein Schieberelement (212), das lösbar mit der vasookklusiven Vorrichtung (210) gekoppelt ist.

18. Vasookklusive Anordnung nach Anspruch 17, ferner umfassend:
eine Ablösevorrichtung (214), die das Schieberelement (212) lösbar mit der vasookklusiven Vorrichtung (210) koppelt.

19. Vasookklusive Anordnung nach Anspruch 18, wobei die Ablösevorrichtung (214) eines von elektrolytisch, mechanischem Verbinder, Erwärmen und Auflösen umfasst.

## Revendications

1. Dispositif médical (210) comprenant :
une partie principale allongée (224) ayant une extrémité proximale (226) et une extrémité distale (228), la partie principale (224) ayant une première rigidité à la flexion, la partie principale (224) ayant plusieurs ouvertures sur une longueur de la partie principale (224) ;
un segment distal atraumatique (230) couplé à l'extrémité distale (228) de la partie principale (224) et s'étendant distalement à partir de celle-ci, le segment distal (230) ayant une deuxième rigidité de flexion, le segment distal (230) ayant une pointe distale (236) ; et
dans lequel la pluralité d'ouvertures de la partie principale est suffisamment grande pour permettre à l'extrémité distale (236) de pénétrer dans les ouvertures et de s'engager dans la partie principale (224);
**caractérisé en ce que**,
le rapport entre la deuxième rigidité de flexion et la première rigidité de flexion est compris entre 0,6 et 0,8, inclusivement, de sorte que le segment distal (230) évite de s'engager dans les ouvertures de la partie principale (224) pendant le déploiement.

2. Dispositif médical de la revendication 1, dans lequel :
la pluralité d'ouvertures s'étend sur au moins 50 % de la longueur de la partie principale (224).

3. Dispositif médical de la revendication 1, dans lequel le rapport entre la deuxième rigidité de flexion et la première rigidité de flexion est compris entre 0,65 et 0,75 inclus.

4. Dispositif médical de l'une des revendications 1 à 3, dans lequel la partie principale allongée (224) comprend une tresse, une maille et un tube ayant une pluralité d'ouvertures.

5. Dispositif médical de la revendication 1, dans lequel le segment distal (230) est une bobine, une bobine hélicoïdale, un tube, une tresse et une tige flexible.

6. Dispositif médical de la revendication 1, comprenant en outre :
une partie proximale atraumatique couplée à l'extrémité proximale de la partie principale et s'étendant de manière proximale à partir de celle-ci.

7. Dispositif médical de la revendication 1, dans lequel le dispositif médical est un dispositif vaso-occlusif.

8. Dispositif vaso-occlusif (210) de la revendication 1,
dans lequel la partie principale (224) comprend une partie tressée allongée (224) comprenant une pluralité de brins allongés tressés ensemble, et le segment distal comprend un segment de bobine distal ; et
et la pluralité d'ouvertures comprend des ouvertures dans la partie tressée (224).

9. Dispositif vaso-occlusif de la revendication 8, dans lequel :
la pluralité d'ouvertures s'étend sur au moins 50 % de la longueur de la partie tressée .

10. Dispositif vaso-occlusif de la revendication 8, comprenant en outre :
un segment de bobine proximale couplé à l'extrémité proximale de la partie tressée et s'étendant de façon proximale à partir de celle-ci.

11. Dispositif vaso-occlusif de la revendication 8, dans lequel la partie tressée (224) a une configuration de délivrance lorsqu'elle est retenue dans un cathéter de délivrance (206) et une configuration déployée lorsqu'elle est libérée d'un cathéter de délivrance (204).

12. Dispositif vaso-occlusif de la revendication 11, dans lequel la configuration de délivrance est une forme sensiblement linéaire, et la configuration déployée est une forme tridimensionnelle ayant une dimension de section transversale d'au moins trois fois la dimension de section transversale de la configuration de délivrance.

13. Dispositif vaso-occlusif de la revendication 8, dans lequel la partie tressée (224) est formée d'un matériau à mémoire de forme.

14. Dispositif vaso-occlusif de la revendication 8, dans lequel la partie tressée (224) est formée de platine, d'un alliage de platine et d'un alliage de platine et de tungstène.

15. Dispositif vaso-occlusif de la revendication 8, dans lequel la partie tressée (224) est formée d'or et d'un alliage d'or.

16. Dispositif vaso-occlusif de la revendication 8, dans lequel la partie tressée (224) est formée d'un alliage platine-or et de nitinol.

17. Assemblage vaso-occlusif (200), comprenant :
le dispositif vaso-occlusif (210) de la revendication 8 ; et
un élément pousseur (212) couplé de manière amovible au dispositif vaso-occlusif (210).

18. Assemblage vaso-occlusif de la revendication 17, comprenant en outre :
un dispositif de détachement (214) reliant de manière amovible l'élément pousseur (212) au dispositif vaso-occlusif (210).

19. Assemblage vaso-occlusif de la revendication 18, dans lequel le dispositif de détachement (214) comprend l'un des éléments suivants : électrolytique, raccord mécanique, chauffage et dissolution.
